# EUROPEAN PATENT APPLICATION

(11) **EP 3 738 548 A1**
(43) Date of publication of application: **18.11.2020**
(21) Application number: 19738428.2
(22) Date of filing: 10.01.2019
(51) Int. Cl.: A61F 2/07

(54) **INTRAVASCULAR PLACEMENT TOOL AND INTRAVASCULAR PLACEMENT SYSTEM**

(30) Priority: 12.01.2018 JP 2018003349
(71) Applicant: Kawasumi Laboratories, Inc., Tokyo 108-6109 (JP)
(72) Inventor: HIDARI, Kentaro, ono-shi, Oita 879-7153 (JP); YUBA, Toshiyasu, ono-shi, Oita 879-7153 (JP)
(74) Representative: Hynell Intellectual Property AB
(86) International application number: PCT/JP2019/000521
(87) International publication number: WO 2019/139077

(57) **Abstract**

Provided is an intravascular placement tool and an intravascular placement system that, when connecting and using multiple intravascular placement tools, enable smooth transport of an intravascular placement tool that is placed later. An intravascular placement tool (stent graft 1) is placed in a first blood vessel (main blood vessel V1) where a second blood vessel (branched blood vessel V2) branches. The intravascular placement tool (1) includes: a tubular placement tool body (graft body 10); and a branching portion (20) that is protrudingly provided on the placement tool body (10) and that can be connected with another intravascular placement tool (second stent graft 2) placed in the second blood vessel (V2). The relative arrangement between the placement tool body (10) and the branching portion (20) is defined on the basis of the relative positional relationship between the first blood vessel (V1) and the second blood vessel (V2).

## Description

### TECHNICAL FIELD

The present invention relates to an intravascular placement tool and an intravascular placement system.

### BACKGROUND ART

Conventionally, as an intravascular placement tool used for treating aortic aneurysm, aortic dissection, or the like caused in an aorta, a stent graft in which a frame portion called a stent is situated on a peripheral face of an artificial blood vessel (graft) is known (e.g. see Patent Document 1).

In stent graft implantation, a plurality of stent grafts may be connected depending on a state of a lesion site. For example, when a stent graft placement region on an aorta includes a branched blood vessel, i.e. when a stent graft is placed in the blood vessel branching portion on the aorta, a branched blood vessel stent graft should be connected to a main blood vessel stent graft for securing a bloodstream toward the branched blood vessel. Hereinafter, a stent graft to be first placed in a blood vessel is referred to as "first stent graft", and a stent graft to be later placed is referred to as "second stent graft".

As described above, when a plurality of stent grafts are used in connection with each other, the first stent graft has a branching portion for connecting the second stent graft. The branching portion is formed e.g. protrudingly from a tube wall of a tubular placement tool main body. The contracted second stent graft having a diameter equal to or larger than an inner diameter of the branching portion is inserted into the branching portion of the first stent graft, and expanded. Then overlapped portions of the second stent graft and the first stent graft are brought into close contact with each other to connect the second stent graft to the first stent graft. The connection state is maintained by an expansion force of the second stent graft on the overlapped part of the first stent graft and the second stent graft.

### PRIOR ART DOCUMENT

### PATENT DOCUMENT

Patent Document 1: Japanese Unexamined Patent Application Publication No. 2009-540930

### SUMMARY OF THE INVENTION

### TECHNICAL PROBLEM

Conventionally, a direction of a branched blood vessel extending from a main blood vessel (direction of the branched blood vessel connecting to the main blood vessel) has not been taken into consideration when forming a branching portion on a first stent graft, and the branching portion has been protrudingly formed perpendicular to a placement tool main body. However, the direction of the branched blood vessel connecting to the main blood vessel is not constantly perpendicular to the main blood vessel. In this case, when a guide wire for transporting a second stent graft is inserted into the branched blood vessel through the branching portion of the first stent graft, a distal end of a guide wire is likely to collide with the blood vessel wall and bend, or to deviate into an area other than the branched blood vessel.

An object of the present invention is to provide an intravascular placement tool and an intravascular placement system that make it possible to smoothly transport an intravascular placement tool to be later placed when using a plurality of intravascular placement tools in connection with each other.

### SOLUTION TO PROBLEM

The intravascular placement tool according to an aspect of the present invention is an intravascular placement tool that is placed in a blood vessel branching portion on a first blood vessel where a second blood vessel (excluding a blood vessel perpendicularly connected to the first blood vessel) branches, the intravascular placement tool including a tubular placement tool main body, and a branching portion that is configured to be connectable with another intravascular placement tool to be placed in the second blood vessel, and is formed protrudingly from the placement tool main body, wherein a relative arrangement between the placement tool main body and the branching portion is defined on the basis of a relative positional relationship between the first blood vessel and the second blood vessel.

The intravascular placement system according to an aspect of the present invention is an intravascular placement system for placing an intravascular placement tool in a blood vessel branching portion on a first blood vessel where a second blood vessel (excluding a blood vessel perpendicularly connected to the first blood vessel) branches, wherein the intravascular placement tool includes a tubular placement tool main body, and a branching portion that is configured to be connectable with another intravascular placement tool to be placed in the second blood vessel, and is formed protrudingly from the placement tool main body, and a relative arrangement between the placement tool main body and the branching portion is defined on the basis of a relative positional relationship between the first blood vessel and the second blood vessel.

### ADVANTAGEOUS EFFECT OF THE INVENTION

The present invention makes it possible to smoothly transport an intravascular placement tool to be later placed when using a plurality of intravascular placement tools in connection with each other.

### BRIEF DESCRIPTION OF THE DRAWINGS

FIG. 1A and FIG. 1B are diagrams illustrating a state that a stent graft according to an embodiment of the present invention is placed in a blood vessel.
FIG. 2 is a perspective view illustrating a first stent graft.
FIG. 3 is a diagram illustrating a connection structure between a graft main body and a branching portion.
FIG. 4A and FIG. 4B are diagrams illustrating a configuration of a stent graft placement system for placing the stent graft in the blood vessel.
FIG. 5A and FIG. 5B are diagrams illustrating a usage form for the first stent graft.

### DESCRIPTION OF THE EMBODIMENT

Hereinafter, an embodiment of the present invention will be explained in detail with reference to the drawings. In this embodiment, a case that stent grafts 1 and 2 are connected and placed in a blood vessel will be explained. The stent graft 1 corresponds to the "intravascular placement tool (intravascular indwelling instrument)" according to the invention, and the stent graft 2 corresponds to the "another intravascular placement tool" in the present invention.

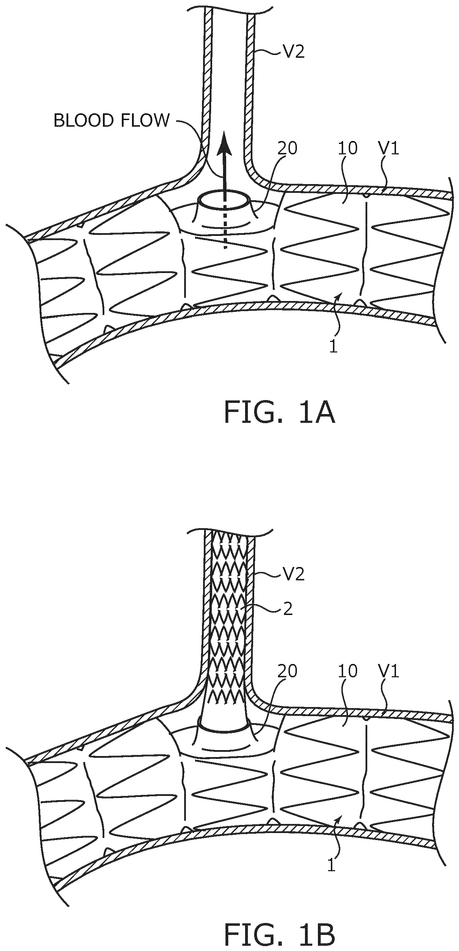

FIG. 1A and FIG. 1B are diagrams illustrating states that the stent grafts 1 and 2 are placed in the blood vessel.

As illustrated in FIG. 1A and FIG. 1B, the stent graft 1 is a main blood vessel stent graft (hereinafter, referred to as "first stent graft 1") placed on a lesion site of an aorta as a main blood vessel V1 (e.g. site having aortic aneurysm or the like). The stent graft 2 is a branched blood vessel stent graft (hereinafter, referred to as "second stent graft 2") placed on a branched blood vessel V2 such as brachiocephalic artery, left common carotid artery, and left subclavian artery, that branches from the main blood vessel V1 in the vicinity of the lesion site.

An outer diameter of the second stent graft 2 in an expanded state is equal to or larger than an inner diameter of a branching portion 20 of the first stent graft 1.

When the first stent graft 1 and the second stent graft 2 are placed in a blood vessel, first, the first stent graft 1 in a contracted state is transported to the lesion site and then expanded, so that the first stent graft 1 is placed in the main blood vessel V1 (see FIG. 1A). Subsequently, the second stent graft 2 in a contracted state is inserted into the branching portion 20, positioned such that the second stent graft 2 and the branching portion 20 are overlapped with a predetermined length, and then expanded, so that the second stent graft is placed in the branched blood vessel V2 (see FIG. 1B). Thereby, on the overlapped part, an inner peripheral face of the first stent graft 1 (branching portion 20) and an outer peripheral face of the second stent graft 2 are brought into close contact with each other, so that the first stent graft 1 (branching portion 20) and the second stent graft 2 are connected to each other. The second stent graft 2 may be transported through the inside of the first stent graft 1 or through the branched blood vessel V2 and inserted into the branching portion 20.

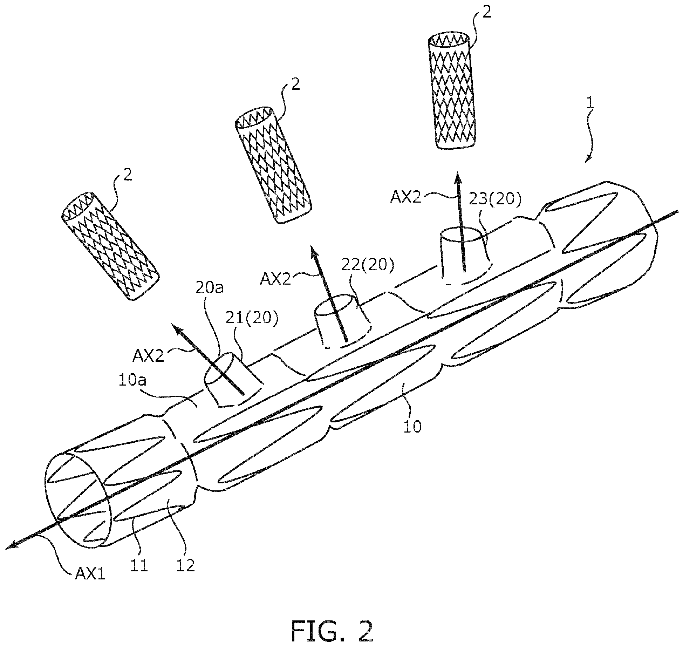

FIG. 2 is a perspective view illustrating the first stent graft 1. FIG. 2 schematically illustrates the expanded first stent graft 1.

As illustrated in FIG. 2, the first stent graft 1 includes a tubular graft main body 10 (placement tool main body) having openings at both ends, and the branching portions 20 formed on a tube wall of the graft main body 10. In this embodiment, although the case that the first stent graft 1 includes three branching portions 21, 22, and 23 is described, the number of the branching portions 20 formed on the graft main body 10 is not particularly limited. Incidentally, the configuration of the second stent grafts 2 is the same as of the graft main body 10.

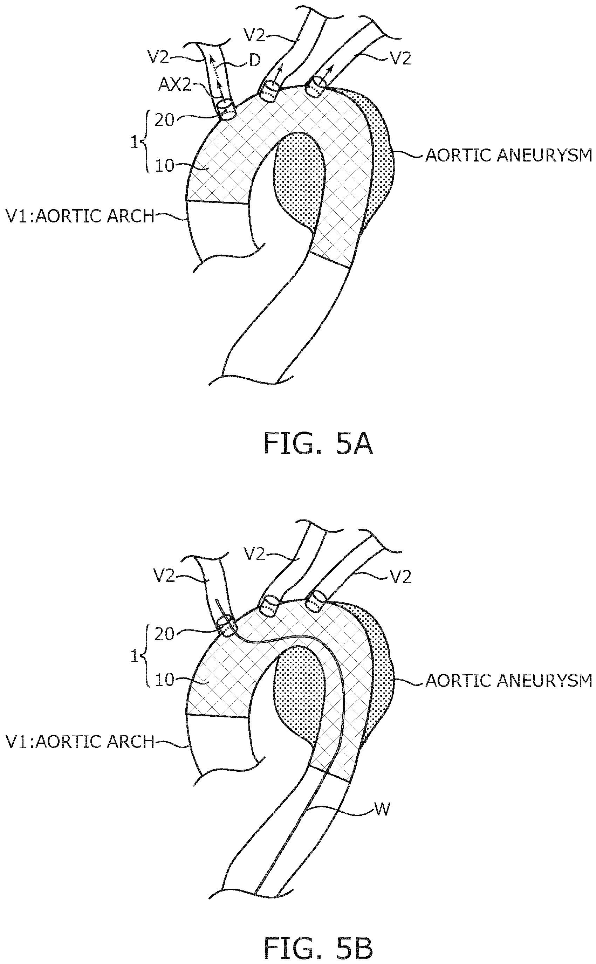

The graft main body 10 has a tubular shape that defines a blood flow path. In this embodiment, although the case that the graft main body 10 has a straight tubular shape is described, the graft main body 10 may have a curved shape depending on a placement site (e.g. a shape corresponding to a shape of a patient's aortic arch (see FIG. 5A, and the like)), or a curved shape along a shape of a blood vessel after the placement.

The graft main body 10 includes a frame portion 11 and a membrane portion 12. The membrane portion 12 is fixed to a peripheral face of the frame portion 11 by suturation using e.g. a suture. Incidentally, the membrane portion 12 may be fixed to the frame portion 11 by a method other than suturation, such as sticking with a tape, adhesion, and welding.

The frame portion 11 is a stiffening member for holding the membrane portion 12 in a predetermined expanded state. The frame portion 11 is e.g. a self-expandable stent frame that is tubularly formed while bending a thin metal wire in a zigzag pattern in axial direction. Incidentally, the frame portion 11 may be made of a material other than metals (e.g. ceramic, resin, or the like).

The frame portion 11 is configured to be transformable from a state of contracting inward in a radial direction to a state of expanding outward in the radial direction to define a tubular flow pass. Examples of a material of the thin metal wire for forming the frame portion 11 include known metals or metal alloys represented by a stainless steel, a Ni-Ti alloy, a titanium alloy, and the like.

The membrane portion 12 is a graft portion that serves as a blood flow path, and situated along a peripheral face of the frame portion 11. Examples of a material for forming the membrane portion 12 include a fluororesin such as polytetrafluoroethylene (PTFE), a polyester resin such as polyethylene terephthalate, and the like.

The graft main body 10 in an expanded state includes a concave portion 10a on which a part of an outer peripheral face of the graft main body 10 is recessed inward in the radial direction. The concave portion 10a includes a flat bottom face, and the branching portions 21 to 23 are arranged in parallel on the bottom face.

The branching portion 20 is formed protrudingly from a tube wall (bottom face of the concave portion 10a) of the graft main body 10 outward in the radial direction of the graft main body 10. The branching portion 20 includes a through-hole 20a communicating with an inner cavity of the graft main body 10. The second stent graft 2 is inserted into and connected to the through-hole 20a of the branching portion 20.

The frame portion 11 is not situated on the branching portion 20. In addition, the branching portion 20 is made of the same material as of the membrane portion 12 of the graft main body 10 and is formed integrally with the graft main body 10. Thereby, the branching portion 20 has such flexibility that e.g. an opening direction can be changed by a blood flow from the main blood vessel V1 to the branched blood vessel V2.

Although not illustrated in the figure, an annular member may be attached to an opening end part of the branching portion 20. The annular member is made of e.g. a high elasticity material such as silicone rubber, and is fixed to the opening end part of the branching portion 20 by thermal welding, ultrasonic welding, pressure bonding, adhesion, suturation, or the like. Incidentally, the annular member may be made of a rubber material other than silicone, or a material other than rubber (e.g. metal, or the like). A molding material of the annular member may include a rubber or the like as a main material, and additionally a radiopaque substance such as barium sulfate as a sub material.

In the conventional stent graft, the branching portion is formed perpendicular to the graft main body regardless of the extending direction of the branched blood vessel, whereas in this embodiment, the branching portion 20 is formed inclinedly with respect to the graft main body 10 depending on an extending direction D of the branched blood vessel V2. Specifically, each of the three branching portions 21 to 23 is situated on the basis of the relative positional relationship between the corresponding branched blood vessel V2 and main blood vessel V1.

That means, the relative arrangement between the graft main body 10 and the branching portion 20 is defined on the basis of the relative positional relationship between the main blood vessel V1 and the branched blood vessel V2.

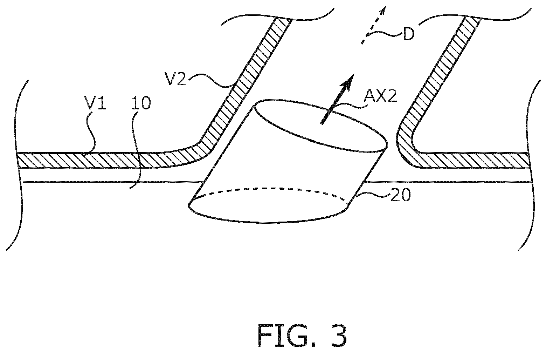

In other words, a connection position and a protruding direction AX2 of the branching portion 20 with respect to the graft main body 10 coincide with the connection position and the extending direction D of the branched blood vessel V2 with respect to the main blood vessel V1 (see FIG. 3). Incidentally, the relative positional relationship between the main blood vessel V1 and the branched blood vessel V2 can be acquired for each patient by a preoperative examination such as computed tomography (CT).

Herein, the protruding direction AX2 of the branching portion 20 is defined by a normal direction of an opening end face of the branching portion 20 (angle between the axial direction AX1 of the graft main body 10 and a normal line). That means, when the protruding direction AX2 of the branching portion 20 with respect to the graft main body 10 coincides with the extending direction D of the branched blood vessel V2, the protruding direction AX2 of the branching portion 20 does not intersect the branched blood vessel V2 in the vicinity of the opening end face of the branching portion 20.

The branching portion 20 has e.g. an obliquely-cut cylindrical shape obtained by obliquely cutting a cylindrical body as illustrated in FIG. 3. In this case, a connection face between the branching portion 20 and the graft main body 10 is elliptic.

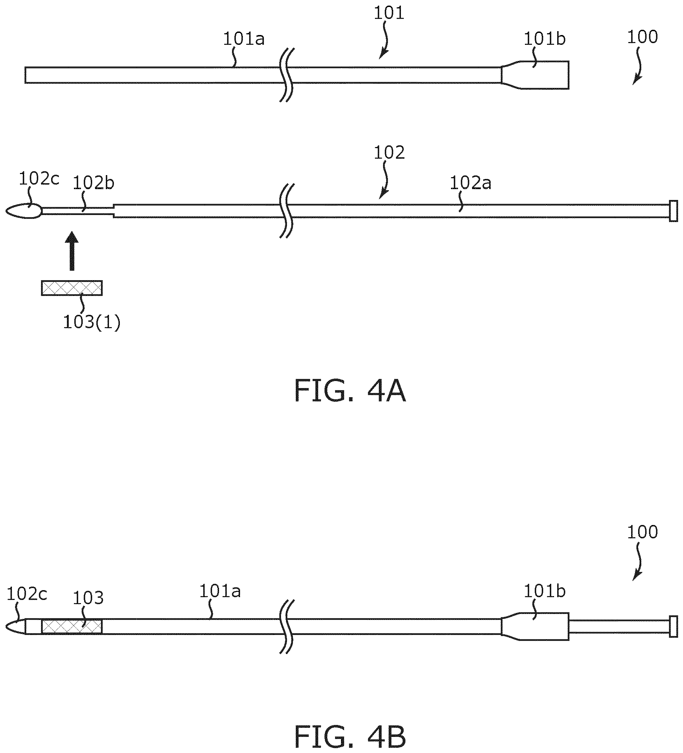

FIG. 4A and FIG. 4B are diagrams illustrating a configuration of a stent graft placement system 100 (intravascular indwelling system) for placing the stent graft in the blood vessel. The first stent graft 1 and the second stent graft 2 can be placed in the blood vessel by using the stent graft placement system 100. FIG. 4A illustrates a state that the stent graft placement system 100 is disassembled. FIG. 4B illustrates a state that the stent graft placement system 100 is assembled.

As illustrated in FIG. 4A and FIG. 4B, the stent graft placement system 100 includes a tubular sheath 101, an inner rod 102 that is situated inside the sheath 101 and configured to be able to advance and retreat along an axial direction of the sheath 101 (longitudinal direction) in the sheath 101, and a stent graft 103 (first stent graft 1, second stent graft 2).

The sheath 101 includes a tubular sheath main body part 101a, and a hub 101b disposed on a base end side (proximal end side) of the sheath main body part 101a. Although not illustrated in the figure, the hub 101b has a nut for fixing the inner rod 102 to the sheath 101 or releasing the fixation.

The sheath 101 is made of a flexible material. Examples of the flexible material include a biocompatible synthetic resin (elastomer) selected from a fluororesin, a polyamide-based resin, a polyethylene-based resin, and a polyvinyl chloride-based resin, and the like, a resin compound obtained by blending other materials into these resins, a multi-layered structure made of these synthetic resins, a complex of these synthetic resins and a metal wire, and the like.

The inner rod 102 has a bar-like rod main body part 102a, a holding part 102b for holding the stent graft 103 in a contracted state, and a distal end tip 102c disposed on a distal end part (distant end part) of the inner rod 102. A diameter of the holding part 102b is set to be smaller than of the rod main body part 102a e.g. by only a thickness of the stent graft 103.

Examples of materials constituting the rod main body part 102a and the holding part 102b include various materials having appropriate hardness and flexibility, such as a resin (plastic, elastomer) and a metal. Examples of a material constituting the distal end tip 102c include various materials having appropriate hardness and flexibility, such as a synthetic resin (elastomer) selected from a polyamide-based resin, a polyurethane-based resin, a polyvinyl chloride-based resin, and the like.

Although not illustrated in the figure, on the rod main body part 102a, the holding part 102b, and the distal end tip 102c, e.g. a lumen through which a guide wire is passed, a lumen through which a trigger wire for expanding the stent graft 103 in a contracted state at a lesion is passed, or the like is formed along an axial direction of the inner rod 102.

As described above, the first stent graft 1 according to this embodiment is an intravascular placement tool placed in the blood vessel branching portion on the main blood vessel V1 (first blood vessel) where the branched blood vessel V2 (second blood vessel) branches. The first stent graft 1 includes the tubular graft main body 10 (placement tool main body), and the branching portion 20 that is configured to be connectable to the second stent graft 2 (another intravascular placement tool) placed in the branched blood vessel V2 and is formed protrudingly from the graft main body 10. The relative arrangement between the graft main body 10 and the branching portion 20 is defined on the basis of the relative positional relationship between the main blood vessel V1 and the branched blood vessel V2.

Thus, since the branching portion 20 is formed depending on the extending direction D of the branched blood vessel V2, when the guide wire W for transporting the second stent graft 2 is inserted into the branched blood vessel V2 through the branching portion 20 of the first stent graft 1, the distal end of the guide wire W hardly collides with the blood vessel wall of the branched blood vessel V2 (see FIG. 5A and FIG. 5B). Thereby, the guide wire W does not deviate into an area other than the branched blood vessel V2, and the second stent graft 2 can be smoothly transported to the branched blood vessel V2.

In particular, even if the graft main body 10 has a configuration with a curved shape corresponding to a curved part (e.g. aortic arch) of the main blood vessel V1, the guide wire for transporting the second stent graft 2 can be properly inserted, because the relative arrangement of the branching portion 20 with respect to the curved graft main body 10 is defined on the basis of the relative positional relationship between the curved part of the main blood vessel V1 and the branched blood vessel V2.

As described above, the invention made by the present inventors has been specifically explained on the basis of the embodiments, but the present invention is not limited to the above embodiments, and can be modified without departing from the gist of the present invention.

For example, the first stent graft 1 and the second stent graft 2 need not be self-expandable, and may be configured to be expanded by an expansion means such as a balloon. Also, the branching portion 20 of the first stent graft 1 may have a tapered shape whose diameter decreases toward the opening end part of the through-hole 20a. This makes it possible to improve adhesiveness between the first stent graft 1 and the second stent graft 2.

Incidentally, in the above embodiment, the configuration that the plurality of second blood vessels V2 are connected to the first blood vessel V1 in the axial direction has been described as an example, but this configuration is merely an example, and the present invention is not limited to this configuration. For example, in a configuration that two or more branched blood vessels are connected to the main blood vessel at positions in substantially the same axial direction, a relative arrangement between the placement tool main body and two or more branching portions corresponding to each branched blood vessel may be defined on the basis of the relative positional relationship of each branched blood vessel with respect to the main blood vessel.

Furthermore, in the above embodiment, the present invention has been explained with reference to the stent graft for thoracic aorta, but the present invention is not limited to this case. For example, although not illustrated in the figure, the present invention can also be applied to stent grafts for abdominal aorta and thoracoabdominal aorta.

In addition, although not explained in detail, the present invention may also be applied to a stent graft intended to be placed on an organ other than blood vessels (e.g. digestive tract, bile duct, or the like).

The embodiments disclosed in this specification should be regarded as examples in all regards and considered to be unrestrictive. The scope of the present invention is stipulated not by the aforementioned explanation but by claims, and intended to include meanings equivalent to claims and all changes within the scope of claims.

Disclosure contents of specifications, figures, and abstracts included in Japanese Patent Application No. 2018-003349 filed on January 12, 2018 are all incorporated in this application.

### DESCRIPTION OF REFERENCE NUMERALS

- 1: First stent graft (intravascular placement tool)
- 10: Graft main body
- 11: Frame portion
- 12: Membrane portion
- 20: Branching portion
- 2: Second stent graft (another intravascular placement tool)
- 100: Stent graft placement system (intravascular placement system)
- AX1: Axial direction of graft main body
- AX2: Protruding direction of branching portion
- D: Extending direction of branched blood vessel
- V1: Main blood vessel (first blood vessel)
- V2: Branching vessel (second blood vessel)

## Claims

1. An intravascular placement tool (1) that is placed in a blood vessel branching portion on a first blood vessel (V1) where a second blood vessel (V2) (excluding a blood vessel perpendicularly connected to the first blood vessel) branches, the intravascular placement tool comprising:
a tubular placement tool main body (10); and
a branching portion (20) that is configured to be connectable with another intravascular placement tool (2) to be placed in the second blood vessel (V2), and is formed protrudingly from the placement tool main body, wherein
a relative arrangement between the placement tool main body (10) and the branching portion (20) is defined on a basis of a relative positional relationship between the first blood vessel (V1) and the second blood vessel (V2).

2. The intravascular placement tool according to claim 1, wherein a protruding direction (AX2) of the branching portion coincides with an extending direction (D) of the second blood vessel on a connection site with the first blood vessel.

3. The intravascular placement tool according to claim 1 or 2, wherein the placement tool main body has a curved shape corresponding to a curved part of the first blood vessel.

4. An intravascular placement system for placing an intravascular placement tool in a blood vessel branching portion on a first blood vessel where a second blood vessel (excluding a blood vessel perpendicularly connected to the first blood vessel) branches, wherein
the intravascular placement tool includes:
a tubular placement tool main body; and
a branching portion that is configured to be connectable with another intravascular placement tool to be placed in the second blood vessel, and is formed protrudingly from the placement tool main body, and
a relative arrangement between the placement tool main body and the branching portion is defined on a basis of a relative positional relationship between the first blood vessel and the second blood vessel.
